(19) Europäisches Patentamt
European Patent Office
Office européen des brevets

(11) Veröffentlichungsnummer: **0 006 187**
**B1**

(12) # EUROPÄISCHE PATENTSCHRIFT

(21) Anmeldenummer: **79101800.5**

(22) Anmeldetag: **07.06.79**

(51) Int. Cl.³: **C 07 B 19/00,**
C 07 C 51/42, C 07 C 57/32,
C 07 C 61/16

(54) **Enantiomerentrennung von chiralen Carbonsäuren**

(30) Priorität: **20.06.78 DE 2826952**

(43) Veröffentlichungstag der Anmeldung:
**09.01.80 Patentblatt 80/01**

(45) Bekanntmachung des Hinweises auf die Patenterteilung:
**10.12.80 Patentblatt 80/25**

(84) Benannte Vertragsstaaten:
**BE CH DE FR GB IT NL**

(56) Entgegenhaltungen:
**DE - A - 2 809 794**
**DE - B - 2 300 325**

**Chemical Abstracts, Band 86, Nr. 7**
**14 Februar 1977**
**(COLUMBUS, OHIO, USA)**
**H. SUGIYAMA et al. "Optical resolution of DL-phenylglycine"**
**Seite 568**

(73) Patentinhaber: **BAYER AG**
**Zentralbereich Patente, Marken und Lizenzen**
**D - 5090 Leverkusen 1**
**Bayerwerk (DE)**

(72) Erfinder: **Naumann, Klaus, Dr.**
**Wolfskaul 2**
**D - 5000 Köln 80 (DE)**

**EP 0 006 187 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Enantiomerentrennung von chiralen Carbonsäuren

Die vorliegende Erfindung betrifft ein neues Verfahren zur Racematspaltung von chiralen Carbonsäuren.

Die Spaltung von racemischen Carbonsäuren in ihre Enantiomeren durch Umsetzung mit optisch aktiven Aminen und Ausnützung der Energieunterschiede, welche sich in unterschiedlichen physikalischen Eigenschaften der so entstandenen diastereomeren Verbindungen äußert, ist eine seit langem gebräuchliche Methode. In der Praxis jedoch ergeben sich vielfältige unvorhersehbare Schwierigkeiten, die eine generelle Uebertragbarkeit der Ergebnisse einer erfolgreichen Racematspaltung selbst auf ähnliche Trennprobleme unmöglich macht. Jedes Trennproblem erfordert seine individuelle Lösung. In diesem Sinne bemerkt auch Elliel in "Stereochemie der Kohlenstoffverbindungen", Verlag Chemie Weinheim 1966, S. 59—99: "Racematspaltung ist nach wie vor eine Kunst".

Es ist technisch von besonderem Interesse die Racemate der für Pyrethroidinsektizide wichtigen Carbonsäuren zu trennen, da die einzelnen Enantiomeren große Wirkungsunterschiede aufweisen. Diese Trennungen erfolgen bis jetzt durch fraktionierte Ausfällung bzw. Kristallisation der Salze mit optisch aktiven Aminen in die jeweiligen Enantiomeren.

Die nachstehende Tabelle gibt einen Überblick über die Versuche zur Lösung der Aufgabe, racemische Carbonsäuren, die für Pyrethroide von Bedeutung sind, in ihre Enantiomeren zu trennen:

| Carbonsäure | | opt. akt. Amin | | Lösungsm. | Literatur |
|---|---|---|---|---|---|
| trans | 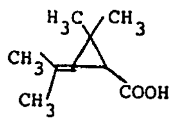 | 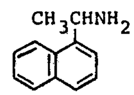 | 1 : 1 | $C_2H_5OH$ | Derwent Basic Abstract Journal (BAJ) 13445 W/08 Japan. Anm. J 4 9 109344 |
| trans | ,, | 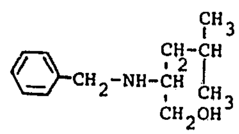 | 1 : 1 | i $C_3H_7OH$ | BAJ 15058 W/09 Japan, Anm. J 4 9092049 |
| trans | ,, | 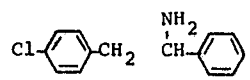 | 1 : 1 | 20% $H_2O$<br>80% $C_2H_5OH$ | BAJ 79586 W/48 Japan. Anm. J 7 503019 |
| trans | ,, | 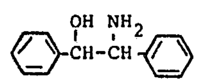 | 1 : 1 | 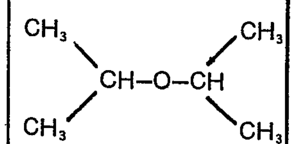 | BAJ 38880 X/21 Japan. Anm. J 5 1041344 |
| trans | ,, | 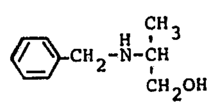 | 1 : 1 | | Agr. Biol. Chem. *37*, 1713–1716 (1973) |

| Carbonsäure | opt. akt. Amin | | Lösungsm. | Literatur |
|---|---|---|---|---|
| cis / trans 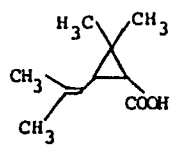 | 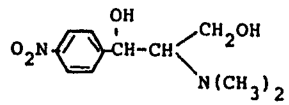 | 1 : 1 | 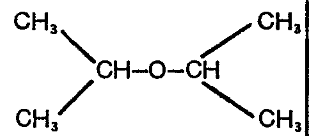 | Franz. Anm. 153 6458 |
| cis ,, | 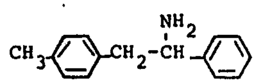 | 1 : 1 | $CH_3COCH_3$ | BAJ 21671 W/13 Japan. Anm. J 4 9125342 |
| trans 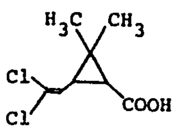 | 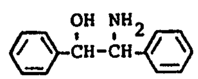 | 1 : 1 | $CH_3OH$ | BAJ 35181 X/19 Japan. Anm. J 5 1036441 |
| trans ,, | 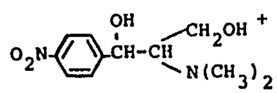 + | 1 : 1 | 50% $H_2O$ 50% $CH_3OH$ | BAJ 85207 Y/48 Japan. Anm. J 5 131953 |
| trans ,, | 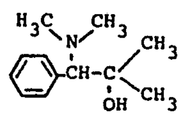 | 1 : 1 | $H_2O$ | BAJ 06690 Y/04 Japan. Anm. J 5 1143647 |
| trans ,, | 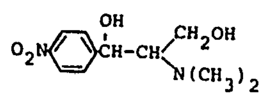 | 1 : 1 | $CH_3COOC_2H_5$ | DE–OS 2439177 |

+ Diese Trennung gelang nicht mit opt. akt. α-Naphthylamin.

| Carbonsäure | opt. akt. Amin | | Lösungsm. | Literatur |
|---|---|---|---|---|
| cis 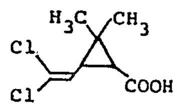 | 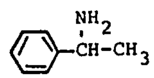 | 1 : 1 | $CH_3COOC_2H_5$ | DE–OS 2549177 |
| 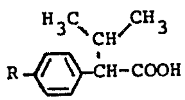 R= $OCH_3$, F, Cl, Br | 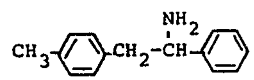 | 1 : 1 | 40% $H_2O$<br>60% $C_2H_5OH$ | BAJ 45194 W/27<br>Japan. Anm.<br>J 5 25 544 |
| 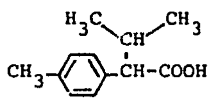 | 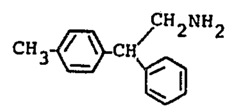 | 1 : 1 | 40% $H_2O$<br>60% $C_2H_5OH$ | BAJ 82419 W/50<br>Japan. Anm.<br>J 5 106 935 |
| 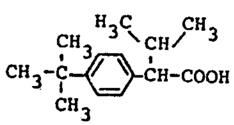 | 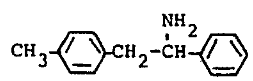 | 1 : 1,2 | 20% $H_2O$<br>80% $C_2H_5OH$ | BAJ 10328 X/06<br>Japan. Anm.<br>J 5 126 635 |

Diese Verfahren halten sich an die oben erwähnte klassische Verfahrensweise, indem äquimolare Mengen Amin und Säure zunächst umgesetzt werden, und dann das entstandene Salz fraktioniert kristallisiert wird. Diese Verfahren sind für eine Anwendung in großem Maßstab umständlich, weil sie z.T. vielfache Kristallisationsstufen verlangen und das erwünschte Enantiomere meist in unbefriedigender Ausbeute ergeben. Es werden optisch aktive Amine erforderlich, welche in größeren Mengen nicht leicht zugänglich sind. Außerdem ist die Rückführung des Amins für einen technischen brauchbaren kontinuierlichen Prozess nur auf komplizierte Weise möglich. So sind diese Verfahren zur Bewältigung des Trennproblems im großen Maßstab nicht geeignet.

Es wurde nun ein Verfahren zur Trennung von Enantiomeren von chiralen Carbonsäuren gefunden, das dadurch gekennzeichnet ist, daß man die Alkalisalze der Säuren mit nur einem Enantiomeren der äquivalenten Menge eines optisch aktiven primären, sekundären oder tertiären Aminsalzes umsetzt, wobei als Reaktionsmedium eine wäßrige schwach alkalische gepufferte Lösung mit einem pH-Wert bei dem die gesamte Säure in der ionisierten Form vorliegt aber noch kein Aminsalz deprotoniert wird, verwendet wird.

Des weiteren wurde gefunden, daß man das aus dem leicht herstellbaren D-Phenylglycin (Derwent BAJ 74855 X/40, Jap.Anm. J 5 1 095—036) glatt zu gewinnende Estersalz, z.B. (—)-Phenylglycinethylesterhydrochlorid (J. biol. Chem. *135*, 91 (1940)) vorteilhaft für die Racematspaltung einsetzen kann.

Bei der erfindungsgemäßen Umsetzung wird selektiv nur ein Enantiomeres als optisch aktives Ammoniumsalz ausgefällt, das andere Enantiomere bleibt als Alkalisalz in Lösung. Mit starken Mineralsäuren werden die enantiomeren Carbonsäuren freigesetzt.

Es ist als ausgesprochen überraschend zu bezeichnen, daß gemäß dem erfindungsgemäßen Verfahren die Trennung der Enantiomeren gelingt, da die für die Trennung entscheidenden Löslichkeitsunterschiede der diastereomeren Salze nicht vorhersehbar war.

Das erfindungsgemäße Verfahren weist eine Reihe von Vorteilen auf. So ist das Verfahren frei von umständ-lichen fraktionierten Kristallisationen. Die gegebenenfalls notwendige Kristallisation der einmal getrennten Enantiomeren erweist sich als einfach, da die eventuell noch vorhandenen racemischen Anteile meist viel schwerer löslich sind als die optisch reinen Verbindungen und so leicht in einem Schritt abgetrennt werden können.

Das erfindungsgemäße Verfahren ist vor allem zur Gewinnung größerer Mengen an reinen Enantiomeren geeignet. Aus den getrennten Salzen können die Enantiomeren in einfacher Weise direkt freigesetzt werden unter leichter Rückführungsmöglichkeit des Amins, wodurch das erfindungsgemäße Verfahren auch leicht kontinuierlich betrieben werden kann.

Verwendet man z.B. 2 Mol (±)trans 2-(2,2-Dichlorvinyl)-3,3-dimethyl-cyclopropancarbonsäure als Ausgangsstoff, wässrige Natriumhydrogencarbonatlösung als alkalisches Medium, 1 Mol (—)Phenylglycinäthylesterhydrochlorid als optisch aktives Amin und Salzsäure als freisetzende Säure, so kann der Reaktionsablauf durch das folgende Formel — schema wiedergegeben werden:

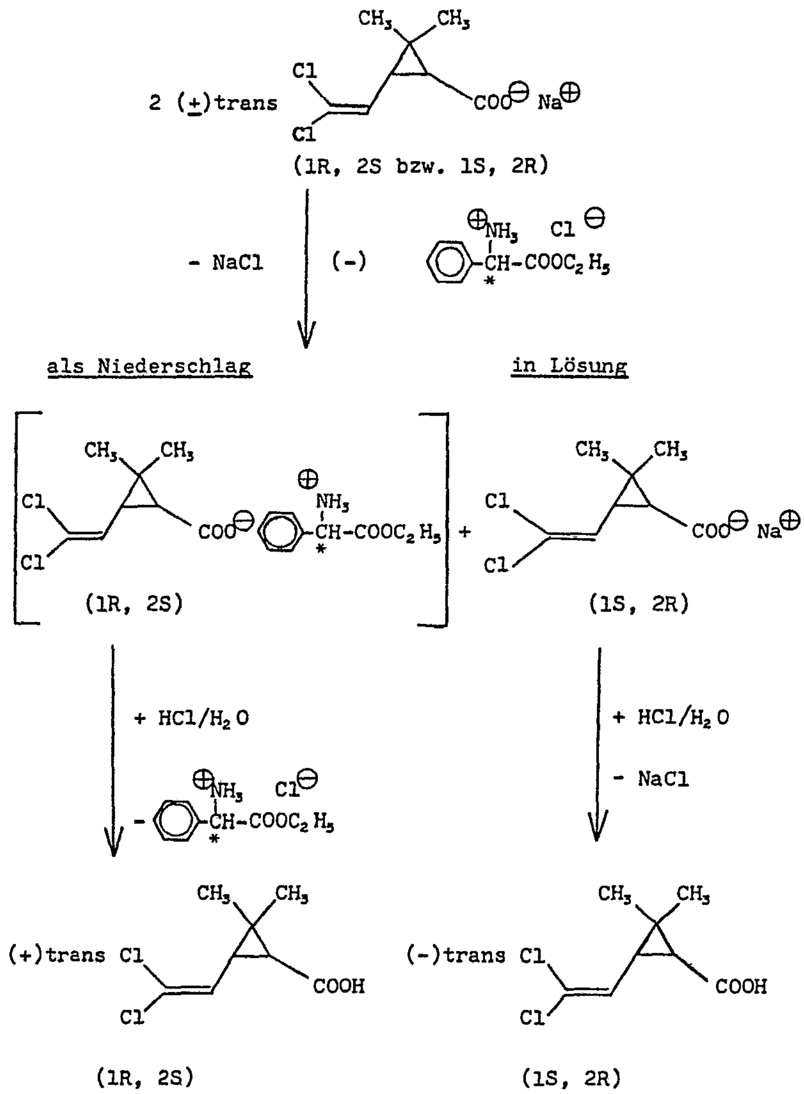


Das erfindungsgemäße Verfahren wird bevorzugt zur Trennung chiraler Carbonsäuren der allgemeinen Formel I verwendet

$$R—COOH \qquad I.$$

in welcher

R für einen substituierten Cyclopropylrest oder für einen gegebenenfalls substituierten 1-Phenyl-2-methyl-propyl (1)-rest steht.

Besonders geeignet ist das erfindungsgemäße Verfahren zur Trennung der Carbonsäuren der allgemeinen Formel I in welcher R für Rest steht

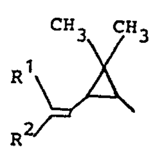

wobei

$R^1$ und $R^2$ gleich oder verschieden sind und für Wasserstoff, Alkyl mit 1—4—C-Atomen, Halogen insbesondere Chlor oder Brom, gegebenenfalls substituiertes Phenyl, oder gegebenenfalls substituiertes Phenylmercapto stehen.

Als Beispiele für die nach dem erfindungsgemäße Verfahren in die Enantiomeren trennbaren Carbonsäuren seien im einzelnen genannt:

(±)cis bzw. (±)trans-2-(2-Methylpropenyl)-3,3-dimethylcyclopropancarbonsäure,

(±)cis bzw. (±)trans-2-(2,2-Dichlorvinyl)-3,3-dimethylcyclopropancarbonsäure,
(±)cis bzw. (±)trans-2-(2,2-Dibromvinyl)-3,3-dimethylcyclopropancarbonsäure,
α-Isopropyl-p-chlorphenylessigsäure,
(±)cis bzw. (±)trans-2-(2-Chlor-2-p-chlorphenyl-vinyl)-3,3-dimethylcyclopropancarbonsäure.

Die als Ausgangsprodukte zu verwendenden racemischen freien Carbonsäuren sind bekannt (vergleiche DE—OS 2 728 150, DE—OS 2 439 177 und die Literatur in der Tabelle). Diese Carbonsäuren werden bevorzugt in Form ihrer Alkalisalze eingesetzt.

Für das erfindungsgemäße Verfahren werden optisch aktive Amine in Form ihrer mineralsauren Salze verwendet. Als Amine seien beispielsweise genannt die Alkylester des Phenylglycins, α-Methylbenzylamin, Phenylethylamin, Phenylethanolamin, 1-Phenyl-2-dimethylamino-1,3-propandiol und Abietylamin.

Das erfindungsgemäße Verfahren wird in Gegenwart von Puffersubstanzen, welche ein konstantes, schwach alkalisches Milieu mit einem pH-Wert, bei dem die gesamte Säure in der ionisierten Form vorliegt aber noch kein Aminsalz deprotoniert wird, aufrechterhalten, durchgeführt. Dafür werden vorzugsweise wäßrige Lösungen anorganischer Salze mehrbasiger Säuren, wie z.B. der Phosphor- und Kohlensäure verwendet, Hierzu gehören vorzugsweise Kaliumcarbonat, Natriumcarbonat, Natriumhydrogencarbonat, Natriumhydrogenphosphat.

Die enantiomeren Carbonsäuren werden nach dem erfindungsgemäßen Verfahren durch Zugabe von starken Säuren in Freiheit gesetzt. Als Säuren kommen dabei vorzugsweise anorganische Säuren wie Salzsäure, Schwefelsäure oder organische Säuren wie Trifluoressigsäure oder Trichloressigsäure in Frage.

Bei der Durchführung des erfindungsgemäße Verfahrens verwendent man pro Mol racemischer Carbonsäure die dem Anteil eines Enantiomeren entsprechende Menge eines optisch aktiven Aminsalzes sowie vorzugsweise mindestens molare Menge der Puffersubstanz. Ein geringer Unterschuß an optisch aktivem Aminsalz ist nicht von Schaden. Die Reaktionspartner werden vorzugsweise zwischen 0°C und 100°C zusammengegeben. Beim Zusammengeben in der Hitze läßt man langsam auskristallisieren, beim Zugeben in der Kälte werden die Komponenten langsam vereinigt, wobei die Fällung gleich eintritt.

Während das Aminsalz des einen Enantiomeren als die schwer lösliche Komponente ausfällt, bleibt das Alkalisalz des anderen Enantiomeren in Lösung. Aus beiden Salzen werden durch Zugabe der notwendigen Menge starker Säure jeweils die freien optisch aktiven Säuren in stark angereicherter bis optisch reiner Form freigesetzt. Die dabei wieder entstehende wäßrige Lösung des optisch aktiven Aminsalzes kann direkt wieder für eine erneute Fällung verwendet werden. Gegebenenfalls kann durch Umkristallisation die optische Reinheit bei nicht ganz getrennten Enantiomeren erhöht werden, wobei die restlichen racemischen Anteile meist als der schwerer lösliche Anteil leicht abtrennbar ist und erneut in die Trennung eingebracht werden kann.

Als Lösungsmittel zum Umkristallisieren eignen sich unpolare Lösungsmittel wie Alkane mit bis zu 10 Kohlenstoffatomen, Petrolether, Cyclohexan, ferner Halogenalkane, wie Tetrachlormethan, aber auch stark polare Lösungsmittel, wie wäßrige Alkohole, Ketone sowie Ether.

In einer besonderen Ausführungsform des erfindungsgemäßen Verfahrens kann gegebenenfalls der restliche Anteil des unerwünschten Enantiomeren in einer durch Ausfällung mit einem optisch aktiven Amin abgetrennten optisch unreinen Carbonsäure nach einem der obigen Fällung analogen Verfahren abgetrennt werden, indem die schwach alkalische wäßrige Lösung der Alkalisalze dieser optisch unreinen Säure mit der der restlichen Menge Enantiomer äquivalenten Menge des gleichen optisch aktiven Amins der der diesmal entgegengesetzten absoluten Konfiguration versetzt wird.

Die nach dem erfindungsgemäße Verfahren erhaltenen reinen, optisch aktiven Carbonsäuren werden für die Herstellung von hochwirksamen Insektiziden vom Pyrethroid-Typ verwendet.

Die folgenden Beispiele illustrieren das erfindungsgemäße Verfahren, ohne eine Einschränkung hinsichtlich der Breite seiner Verwendbarkeit anzugeben.

Beispiel 1

Zu I Mol (±)trans-2-(2,2-Dichlorvinyl)-3,3-dimethylcyclopropancarbonsäure und I Mol Natriumcarbonat in 10 ltr. Wasser wird bei 20°C unter Rühren eine Lösung von 0,5 Mol (—)-Phenylglycinethylester-hydrochlorid 92% optisch rein ($[\alpha]_D^{20}$ = —91°C (1 %ige Lösung in Wasser)) in 1000 ml Wasser zugetropft. Der entstehende Niederschlag wird scharf abgesaugt, mit kaltem Wasser gewaschen und durch Zugabe von 1000 ml 0,25 molarer Schwefelsäure und 1000 ml Ether bei 20° zersetzt. Die organische Phase wird abegtrennt, getrocknet und eingeengt. Man erhält in 95 %iger Ausbeute (+)-trans-2-(2,2-Dichlorvinyl)-3,3-dimethylcyclopropancarbonsäure mit einer optischen Reinheit von 87%. Durch Umkristallisation aus Petrolether und nach Abtrennung des restlichen schwerer löslichen Racematanteils erhält man die optisch reine (+)-trans-Säure mit einem Drehwert von $[\alpha]_D^{20}$ = +36,0° (1 %ige Lösung in Chloroform).

Aus der wäßrigen Phase erhält man bei entsprechender Aufarbeitung die (—)trans-2-(2,2-Dichlorvinyl)-3,3-dimethylcyclopropancarbonsäure mit einem Drehwert von $[\alpha]_D^{20}$ = —36,0° (1 %ige Lösung in Chloroform).

Beispiel 2

In gleicher Weise verfährt man mit der $\alpha$-Isopropyl-p-chlorphenylessigsäure. Unter Verwendung eines 87% optisch reinen (—)-Phenylglycinethylestersalzes erhält man einen Niederschlag der das (—)-Enantiomere der Carbonsäure mit 75% optischer Reinheit enthält. $[\alpha]_{20}^{D} = -36°$ (C=1, $CHCl_3$) (der Drehwert der optisch reinen (—)-S-$\alpha$-Isopropyl-p-chlorphenylessigsäure beträgt laut DE—OS 2737 297 $[\alpha]_{20}^{D} -48{,}3°$ ($CHCl_3$)). Aus Petrolether kristallisiert zuerst racemische Säure, in Lösung bleibt eine optisch stark angereicherte Säure.

## Patentansprüche

1. Verfahren zur Trennung von Enantiomeren von chiralen Carbonsäuren, dadurch gekennzeichnet, daß man die Alkalisalze der Säuren mit der nur einem Enantiomeren äquivalenten Menge eines optisch aktiven primären, sekundären oder tertiären Aminsalzes umsetzt, als Reaktionsmedium eine wäßrige schwach alkalische gepufferte Lösung mit einem pH-Wert bei dem die gesamte Säure in der ionisierten Form vorliegt, aber noch kein Aminsalz deprotoniert wird, verwendet wird.

2. Verfahren gemäß Anspruch 1, dadurch gekennzeichnet, daß als optisch aktives Aminsalz (—)-Phenylglycinethylesterhydrochlorid verwendet wird.

## Revendications

1. Procédé de séparation d'énantiomères d'acides carboxyliques du type chiral, caractérisé en ce qu'on fait réagir les sels alcalins des acides avec la quantité, qui n'équivaut seulement qu'à un énantiomère, d'un sel d'amine optiquement actif primaire, secondaire ou tertiaire, en utilisant comme milieu de réaction une solution aqueuse faiblement alcaline tamponnée ayant une valeur de pH à laquelle tout l'acide se présente sous la forme ionisée sans qu'il n'y ait toutefois pas encore de déprotonisation du sel d'amine.

2. Procédé selon la revendication 1, caractérisé en ce qu'on utilise comme sel d'amine optiquement actif du chlorhydrate d'ester éthylique de (—)-phénylglycine.

## Claims

1. Process for the separation of enantiomers of chiral carboxylic acids, characterised in that the alkali metal salts of the acids are reacted with an amount of an optically active primary, secondary or tertiary amine salt which is equivalent to only one enantiomer, and an aqueous, weakly alkaline buffered solution with a pH value at which the entire acid is present in the ionised form but at which no amine salt is yet deprotonated is used as the reaction medium

2. Process according to Claim 1, characterised in that (—)-phenylglycine ethyl ester hydrochloride is used as the optically active amine salt.